Europäisches Patentamt

European Patent Office

Office européen des brevets

(11) Numéro de publication: **0 023 862**
**A1**

(19)

(12) # DEMANDE DE BREVET EUROPEEN

(21) Numéro de dépôt: **80401119.5**

(22) Date de dépôt: **29.07.80**

(51) Int. Cl.³: **G 01 R 13/20,** A 61 B 5/04, G 01 R 13/30

(30) Priorité: **07.08.79 FR 7920155**

(43) Date de publication de la demande: **11.02.81 Bulletin 81/6**

(84) Etats contractants désignés: **AT BE CH DE GB IT LI LU NL SE**

(71) Demandeur: **CARDIOFRANCE - COMPAGNIE FRANCAISE D'ELECTROCARDIOLOGIE, 73, route de Neuilly, F-93160 Noisy Le Grand (FR)**

(72) Inventeur: **Buffet, Jacques, 28, Avenue Thiers, F-93340 Le Raincy (FR)**

(74) Mandataire: **Derambure, Christian, Cabinet BUGNION ASSOCIES SARL 116, boulevard Haussmann, F-75008 Paris (FR)**

(54) **Oscilloscope enregistreur pour afficher des échelles de temps en combinaison avec un signal cyclique.**

(57) Ils comportent des moyens (H, L, T) propres à permettre l'enregistrement en sens horizontal d'une échelle de durée déterminée et relative, des moyens (H2) propres à permettre l'enregistrement en sens vertical d'une échelle de temps réel et des moyens (H1) propres à permettre l'enregistrement en déflection vertical d'un signal d'étalonnage.

L'invention est applicable à un oscilloscope pour la reproduction d'un électrocardiogramme sous la forme d'un «contourogramme» de WEBB et ROGERS.

- 1 -

Perfectionnements aux oscilloscopes enregistreurs

L'invention concerne des perfectionnements aux oscilloscopes enregistreurs pour la reproduction chronologique, compacte, en continu, par exemple sous la forme d'un "contourogramme" de WEBB et ROGERS, d'un enregistrement de longue durée
d'un signal d'un phénomène cyclique - en particulier celui
du cycle cardiaque tel qu'un électrocardiogramme (ou ECG).

On connaît déjà le principe du "contourogramme" développé
par WEBB et ROGERS qui consiste à présenter l'enregistrement
de l'ECG de manière que les cycles soient superposés et rapprochés, chaque cycle partant d'un repère déterminé, par
exemple l'onde R de l'ECG. Cette méthode permet de bénéficier de tous les avantages de l'enregistrement électrocardiographique continu et de longue durée sans avoir les inconvénients inhérents qui sont essentiellement la longueur
très importante de l'enregistrement et la difficulté de lecture de ceux-ci notamment en ce qui concerne certaines affections.

On connaît également un oscilloscope enregistreur plus spécialement destiné à mettre en oeuvre cette technique de la
"contourographie" qui comporte une sortie de déflection verticale et une sortie de modulation de brillance auxquelles
est associé le signal ECG, soit directement, soit indirectement par l'intermédiaire d'un premier amplificateur à bande
passante spécifique ; un second amplificateur à bande passante spécifique ayant un étage de synchronisation à seuil
réglable recevant en entrée le signal ECG en vue de détecter un événement déterminé commun à chaque cycle de l'ECG par exemple une onde R, associée à une base de temps à laquelle est associée une commande de lumière, réglable selon d'une part la vitesse de balayage horizontal et d'autre part la vitesse de lecture du signal ECG et comportant une sortie de balayage horizontal ; et des moyens d'enregistrement photographique du spot, à défilement régulier, soit permanent avec ou sans base de temps, soit intermittent entre les balayages.

Cependant, un tel oscilloscope enregistreur présente l'inconvénient d'être d'un usage mal commode pour le praticien étant donné que l'échelle de durée en sens horizontal permettant d'apprécier notamment la période du complexe QRS n'apparaît pas ; la matérialisation de cette durée par un segment étant, toute chose égale par ailleurs, variable et fonction de la vitesse de lecture de l'ECG. D'autre part, l'oscilloscope enregistreur connu présente l'inconvénient de ne pas avoir de façon explicite et matérielle une échelle des temps réel en sens vertical ce qui ne permet donc pas au praticien de déterminer de façon commode l'heure effective à laquelle un événement spécifique s'est produit. Enfin, les oscilloscopes enregistreurs connus ne résolvent pas de façon satisfaisante le problème de l'étalonnage en déflection vertical.

La présente invention a pour but d'apporter un remède à ces difficultés. L'invention, telle qu'elle est caractérisée dans les revendications, résout ces difficultés en proposant un oscilloscope enregistreur pour la reproduction chronologique, compacte, en continu, par exemple sous la forme d'un "contourogramme" d'un enregistrement de longue durée d'un signal d'un phénomène cyclique - en particulier celui du cycle cardiaque tel qu'un ECG - qui comporte une sortie de déflection verticale et une sortie de modulation de brillance auxquelles est associé le signal ECG, soit directement, soit indirectement par l'intermédiaire d'un premier amplificateur à base passante spécifique ; un second amplificateur à base passante spécifique ayant un étage de synchronisation à seuil réglable recevant en entrée le signal ECG en vue de détecter un événement déterminé commun à chaque cycle de l'ECG, par exemple une onde R, associée à une base de temps à laquelle est associée une commande de lumière, réglable selon d'une part la vitesse de balayage horizontal et d'autre part la vitesse de lecture du signal ECG et comportant une sortie de balayage horizontal ; et des moyens d'enregistrement photographique du spot à défilement régulier, soit permanent avec ou sans base de temps, soit intermittent entre les balayages. En outre, cet oscilloscope enregistreur comporte, en combinaison, des moyens propres à permettre l'enregistrement en sens horizontal d'une échelle de durée déterminée et relative ; des moyens propres à permettre l'enregistrement en sens vertical d'une échalle de temps réel et des moyens propres à permettre l'enregistrement en déflection verticale d'un signal d'étalonnage.

Les avantages obtenus grâce à l'invention consistent essentiellement en ce que le praticien peut utiliser le "contourogramme" de façon beaucoup plus commode qu'auparavant puisque celui-ci comporte une échelle de durée, une échelle de temps réel et un étalonnage. Conséquemment, il s'ensuit des risques d'erreurs considérablement diminués. Ces avantages sont en outre obtenus sans complications excessives de l'appareil.

L'invention est plus particulièrement décrite en relation avec un électrocardiogramme (ECG). Il va de soi cependant que tout autre phénomène cyclique est intéressé par les perfectionnements apportés aux oscilloscopes enregistreurs qui permettent l'enregistrement du signal de ce phénomène.

Dans ce qui suit, l'invention est exposée plus en détail à l'aide de dessins représentant deux modes d'exécution possibles.

La figure 1 représente un bloc diagramme schématisant un oscilloscope enregistreur conformément à une première variante de la présente invention.;
La figure 2 représente un bloc diagramme schématisant un oscilloscope enregistreur conformément à une seconde variante de la présente invention.

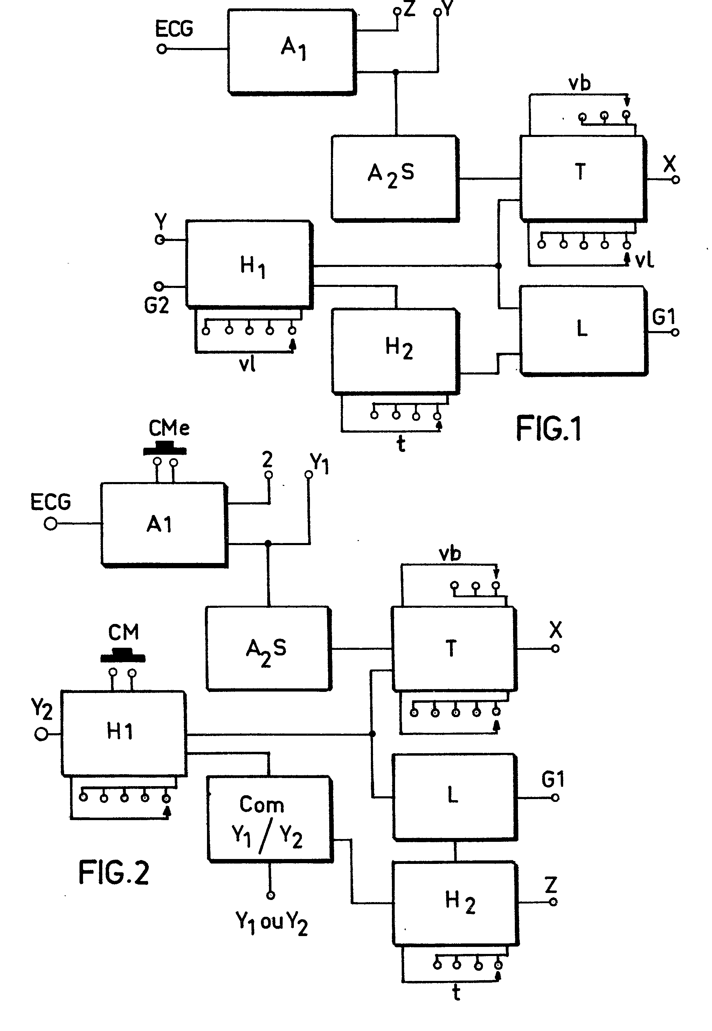

L'oscilloscope enregistreur pour la reproduction chronologique, compacte, en continu, sous la forme d'un "contourogramme" d'un électrocardiogramme (ou ECG) selon l'invention et la figure 1 comporte une sortie de déflection verticale Y et une sortie de modulation de brillance Z auxquelles est associée l'entrée du signal ECG, par l'intermédiaire d'un premier amplificateur A1 à bande passante spécifique notamment 0,2 Hz à 100 Hz environ, à gain ajustable , éventuellement pourvu de filtres haute fréquence et basse fréquence.

En variante toutefois, le signal ECG peut être directement appliqué aux sorties Z et Y dès lors que ce signal a subi une amplification préalable (par exemple dans un lecteur à cassette).

Un second amplificateur A2 à bande passante spécifique , ayant un étage de synchronisation à seuil réglable, A2S reçoit en entrée le signal ECG amplifié par l'amplificateur A1. L'objet de l'amplificateur A2S est de détecter un événement commun à chaque cycle de l'ECG par exemple une onde R qui va servir de base commune pour tous les cycles successifs. L'amplificateur A2S comporte un ensemble de filtres réglables en fonction de la vitesse $v_1$ de lecture du signal ECG de manière que la sécurité attachée à la détection de l'onde R soit aussi élevée que possible sans risque par exemple de retenir des ondes parasites.

L'oscilloscope enregistreur comporte une base de temps T
à laquelle est associée une commande de lumière L . Cette
base de temps T est associée à l'amplificateur A2S et comporte une sortie de balayage horizontal X. La base de temps
T est réglable selon d'une part la vitesse de balayage horizontal $vb$ et d'autre part la vitesse de lecture du signal
. ECG soit $vl$.

Par exemple, l'oscilloscope enregistreur comporte trois vitesses de balayage horizontal $vb$ respectivement 25 mm/s.
50 mm/s. et 100 mm/s. Il peut comporter en outre cinq vitesses de lecture du signal ECG, $vl$ qui correspondent par rapport à la vitesse d'enregistrement de ce signal à un coefficient multiplicateur respectivement de 1 (vitesse de lecture
égale à la vitesse d'enregistrement), 25, 60, 100 et 120.
La commande lumière L comporte en sorite une gate G1 telle
que le spot soit éteint pendant son mouvement de retour horizontal.

Suivant l'invention, l'oscilloscope enregistreur comporte en
combinaison des moyens propres à permettre l'enregistrement
en sens horizontal d'une échelle de. durée déterminée et
relative ; des moyens propres à permettre l'enregistrement
en sens vertical d'une échelle de temps réel et des moyens
de déflection verticale d'un signal d'étalonnage. Dans la
forme d'exécution possible illustrée par la figure 1, ces
moyens sont combinés pour intervenir simultanément c'est à
dire que sur le "contourogramme" apparaîtra en sens vertical
correspondant au défilement du support d'enregistrement du
spot, une échelle de temps réel. Pour chaque marque de
cette échelle de temps réel apparaîtra sur l'axe horizontal
une échelle de durée relative et déterminée. Enfin, la
marque correspondant à cette échelle des durées déterminera
le signal d'étalonnage.

Dans la forme d'exécution illustrée par la figure 1, l'oscilloscope enregistreur est à trace unique et l'enregistrement de l'échelle de durée intervient sur la sortie Y, l'ECG étant inhibé via une gate G2. Dans cette forme d'exécution, l'oscilloscope enregistreur comporte une horloge H1 synchrone du balayage, donc associée à la base de temps via un étage de synchronisation. Cette horloge H1 est réglable en fonction de la vitesse de lecture $v1$. Le signal est donc appliqué à la sortie Y et permet de tracer sur le "contourogramme" des repères espacés correspondant à une durée déterminée et relative par exemple 100 ms. En outre l'importance de la déflection verticale est telle que chaque repère correspond à une valeur d'étalonnage par exemple mV d'amplitude. Bien entendu, l'ECG n'apparaît pas lorsque l'échelle de durée apparaît.

En ce qui concerne les moyens propres à permettre l'enregistrement en sens vertical d'une échelle de temps réel, ils comprennent une horloge H2 pilotant l'horloge H1 et à laquelle sont associés des diviseurs permettant de choisir l'une de plusieurs possibilités de temps $t$ par exemple quatre temps respectivement 15 mn. ; 30 mn. , une heure et huit heures associée également au balayage horizontal notamment à la commande lumière par un étage de synchronisation et ayant, en sortie la modulation de brillance 2.

Dans la variante illustrée par la figure 2, l'oscilloscope enregistreur est à deux traces et comporte donc deux sorties de déflection verticale respectivement Y1 et Y2. A l'une des sorties Y1 par exemple est associé le signal ECG tandis qu'à l'autre sortie Y2 sont associés les moyens propres à permettre l'enregistrement en sens horizontal d'une échelle de durée déterminée relative et propre à permettre l'enregistrement en sens vertical d'une échelle de temps réel. Les deux horloges H1 et H2 sont alors associées à un dispositif de commutation COMY 1 / Y2 qui comporte une sortie correspondant à Y1 ou Y2. Dans cette forme d'exécution, il est prévu une commande manuelle CM de l'horloge H1 c'est à dire des moyens propres à permettre l'enregistrement en sens horizontal d'une échelle de durée déterminée et relative. C'est dire que cette échelle apparaît en tout endroit souhaité du "contourogramme" à la demande même de l'opérateur. Au contraire et bien entendu, l'échelle des temps réels apparaît en permanence. Egalement, dans cette forme d'exécution, les moyens propres à permettre l'enregistrement en déflection verticale d'un signal d'étalonnage sont à commande manuelle CME et associés directement à l'amplificateur A1. Dans ce cas, cet étalonnage intervient sur la sortie Y1 par exemple en début de "contourogramme".

Naturellement, il va de soi que l'invention peut faire encore l'objet de nombreuses autres formes d'exécution.

Revendications de brevet.

1. Oscilloscope enregistreur pour la reproduction chronologique, compacte, en continu, par exemple sous la forme d'un "contourogramme" de WEBB et ROGERS, d'un enregistrement de longue durée d'un signal d'un phénomène cyclique - en particulier celui du cycle cardiaque tel qu'un électrocardiogramme (ECG) - qui comporte une sortie de déflection verticale et une sortie de modulation de brillance auxquelles est associé le signal ECG soit directement, soit indirectement par l'intermédiaire d'un premier amplificateur à bande passante spécifique ; un second amplificateur à bande passante spécifique ayant un étage de synchronisation à seuil réglable recevant en entrée le signal ECG en vue de détecter un événement déterminé commun à chaque cycle de l'ECG par exemple une onde R, associée à une base de temps à laquelle est associée une commande de lumière, réglable selon d'une part la vitesse de balayage horizontal et d'autre part la vitesse de lecture du signal ECG et comportant une sortie de balayage horizontal X, et des moyens d'enregistrement photographique du spot à défilement régulier, soit permanent avec ou sans base de temps, soit intermittent entre les balayages, caractérisé par le fait qu'il comporte en outre en combinaison des moyens propres à permettre l'enregistrement en sens horizontal d'une échelle de durée déterminée et relative, des moyens propres à permettre l'enregistrement en sens vertical d'une échelle de temps réel ; et des moyens propres à permettre l'enregistrement en déflection verticale d'un signal d'étalonnage.

2. Oscilloscope enregistreur suivant la revendication 1, caractérisé par le fait que les moyens propres à permettre l'enregistrement en sens horizontal d'une échelle de durée déterminée et relative comprennent une horloge H1 associée à la base de temps T et à la commande lumière L par un étage de synchronisation, la dite horloge pouvant être réglée selon la vitesse de lecture vl du signal ECG.

3. Oscilloscope enregistreur suivant l'une quelconque des revendications 1 et 2, caractérisé par le fait que les moyens propres à permettre l'enregistrement en sens vertical d'une échelle de temps réel comprennent une horloge en temps réel suivie de diviseurs H2 associée à la commande lumière par un étage de synchronisation, réglable selon une échelle de temps t , associée en sortie à la modulation de brillance Z.

4. Oscilloscope enregistreur suivant l'une quelconque des revendications 1 à 3, caractérisé par le fait qu'il est à trace unique, les moyens propres à permettre l'enregistrement en sens horizontal d'une échelle de durée déterminée et relative fonctionnant en synchronisme avec les moyens propres à permettre l'enregistrement en sens vertical d'une échelle de temps réel, les moyens pour permettre l'enregistrement en déflection verticale d'un signal d'étalonnage fonctionnant également en synchronisme avec des moyens d'enregistrement de l'échelle de durée.

5. Oscilloscope enregistreur suivant l'une quelconque des revendications 1 à 3, caractérisé par le fait qu'il est à deux traces, l'une correspondant au signal ECG et l'autre aux échelles de temps, les deux horloges H1 et H2 étant associées par l'intermédiaire d'une commutation.

1/1

0023862

FIG.1

FIG.2

**Office européen des brevets**

# RAPPORT DE RECHERCHE EUROPEENNE

00237862

EP 80 40 1119

| DOCUMENTS CONSIDERES COMME PERTINENTS | | | CLASSEMENT DE LA DEMANDE (Int. Cl. 3) |
|---|---|---|---|
| Catégorie | Citation du document avec indication, en cas de besoin, des parties pertinentes | Revendication concernée | |
| | <u>FR - A - 975 440</u> (COMPAGNIE FRANCAISE THOMSON-HOUSTON)<br><br>* Page 1, colonne de gauche, lignes 22-40; page 2, colonne de gauche, lignes 1-31; page 3, colonne de gauche, lignes 16-18; page 4, colonne de gauche, lignes 5-11; figures 1,2 *<br>-- | 1,2,5 | G 01 R 13/20<br>A 61 B 5/04<br>G 01 R 13/30 |
| | <u>US - A - 3 267 933</u> (W. MILLS et al.)<br><br>* Colonne 2, lignes 28-39; figures 1,3 *<br>-- | 1 | **DOMAINES TECHNIQUES RECHERCHES (Int. Cl. 3)** |
| | IEEE TRANSACTIONS ON BIO-MEDICAL ENGINEERING, vol. BME-17, no. 4, octobre 1970 NEW YORK (US) D. P. GOLDEN et al.: "Real time contourography - An improved electrocardiographic display", pages 296-301<br><br>* En entier *<br>-- | 1 | A 61 B 5/04<br>G 01 R 13/20<br>13/30<br>13/32<br>G 01 D 9/28<br>G 01 R 13/22<br>13/26<br>19/24 |
| A | <u>US - A - 3 638 066</u> (T. PAINE et al.)<br><br>* Résumé; figure 1 *<br>-- | 1 | **CATEGORIE DES DOCUMENTS CITES** |
| A | IEEE SPECTRUM, juin 1966 NEW YORK (US) G.N. WEBB et al.: "The contourograph", pages 77-87<br><br>* En entier *<br>-- | | X: particulièrement pertinent<br>A: arrière-plan technologique<br>O: divulgation non-écrite<br>P: document intercalaire<br>T: théorie ou principe à la base de l'invention<br>E: demande faisant interférence<br>D: document cité dans la demande<br>L: document cité pour d'autres raisons |
| | | | &: membre de la même famille, document correspondant |

X Le présent rapport de recherche a été établi pour toutes les revendications

| Lieu de la recherche | Date d'achèvement de la recherche | Examinateur |
|---|---|---|
| La Haye | 09.09.1980 | GALLO |

**OEB Form 1503.1  06.78**